(19) Europäisches Patentamt
European Patent Office
Office européen des brevets

(11) **EP 3 456 702 B1**

(12) **FASCICULE DE BREVET EUROPEEN**

(45) Date de publication et mention
de la délivrance du brevet:
**08.07.2020 Bulletin 2020/28**

(51) Int Cl.:
**C07C 1/24** *(2006.01)*          **C07C 5/27** *(2006.01)*
**C07C 11/09** *(2006.01)*         **C07C 11/08** *(2006.01)*

(21) Numéro de dépôt: **18193532.1**

(22) Date de dépôt: **10.09.2018**

(54) **PROCEDE DE DESHYDRATATION DES ALCOOLS EN OLEFINES COMPRENANT LE RECYCLAGE DES ALCOOLS**

VERFAHREN ZUR DEHYDRATISIERUNG VON ALKOHOLEN ZU OLEFINEN, DAS EIN RECYCLING DER ALKOHOLE BEINHALTET

METHOD FOR DEHYDRATING ALCOHOLS TO OLEFINS COMPRISING THE RECYCLING OF ALCOHOLS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.09.2017 FR 1758667**

(43) Date de publication de la demande:
**20.03.2019 Bulletin 2019/12**

(73) Titulaires:
• **IFP Energies nouvelles**
  **92852 Rueil-Malmaison Cedex (FR)**
• **Total Research & Technology Feluy**
  **7181 Seneffe (BE)**

(72) Inventeurs:
• **DROBYSHEV, Kirill**
  **92500 RUEIL MALMAISON (FR)**
• **COUPARD, Vincent**
  **69100 VILLEURBANNE (FR)**
• **GABELLE, Jean-Christophe**
  **38920 CROLLES (FR)**
• **NESTERENKO, Nikolai**
  **1400 NIVELLES (BE)**
• **THILLE, Christophe**
  **1982 Weerde (BE)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 3 162 763          WO-A1-2011/085223**
**WO-A1-2016/046242**

# Description

## DOMAINE TECHNIQUE DE L'INVENTION

La présente invention concerne un procédé de déshydratation des alcools en oléfines comprenant une étape améliorée de récupération de l'alcool non réagi. La charge du procédé peut être obtenue par des procédés chimiques ou par des procédés fermentaires.

Les alcènes obtenus, en particulier l'isobutène, le butène-1 et les butènes-2, présentent un intérêt important dans le domaine de l'industrie pétrochimique et de la synthèse organique.

## ART ANTÉRIEUR

Le document EP 2348 005 décrit la déshydratation d'alcools contenant de 2 à 10 atomes de carbone en l'oléfine correspondante sur un catalyseur zéolithique FER de ratio atomique Si/Al inférieur à 100. La vitesse spatiale horaire massique (Weight Hourly Space Velocity selon la dénomination anglaise, ou WHSV) par rapport à l'alcool est d'au moins 4 h$^{-1}$ et la température de 320 à 600°C.

Le document WO 2011/113834 décrit la déshydratation et l'isomérisation squelettale simultanée de l'isobutanol en présence de catalyseurs silicates cristallins, à taille de canaux moyenne (10MR) déaluminisés ou non, modifiés au phosphore ou non, du groupe FER, MWW, EUO, MFS, ZSM-48, MTT, MFI, MEL ou TON ayant un ratio Si/Al supérieur à 10, tamis moléculaires silicoaluminophosphates du groupe AEL, ou silice-, zircone-, titane- ou fluor-alumine sur catalyseurs zéolithiques. La PPH (ratio du débit massique de charge sur la masse de catalyseur, correspondant à la WHSV) par rapport à l'alcool est d'au moins 1 h$^{-1}$ et la température de 200 à 600°C. La proportion maximale atteinte en n-butènes dans les butènes est de 58.4% à 375°C à forte PPH (12.6 h$^{-1}$) sur une zéolithe FER en poudre de Si/Al 33.

WO 2016/046242 décrit un procédé de déshydratation isomérisante en présence d'un catalyseur comprenant une zéolithe comprenant au moins une série de canaux dont l'ouverture est définie par un anneau à 8 atomes d'oxygène (8MR), ledit catalyseur étant préalablement coké in-situ ou ex-situ, de manière à produire un effluent de déshydratation et comprenant des étapes de compression, chauffe, réaction, refroidissement, décantation, distillation.

Afin de maintenir la cible en conversion, la température moyenne de réaction est augmentée, induisant des baisses de sélectivité. La récupération et le recyclage des espèces réactives non réagies est donc de prime importance pour la viabilité du procédé.

La demanderesse a découvert un arrangement particulier du procédé de déshydratation des alcools permettant une meilleure récupération et recyclage de l'alcool non réagi, permettant ainsi une meilleure conversion globale.

## OBJET ET INTÉRÊT DE L'INVENTION

L'invention concerne un procédé de déshydratation isomérisante d'une charge comprenant de 40 à 100% poids d'alcool primaire substitué en position 2 par un groupement alkyl comprenant au moins les étapes suivantes :

a) Mise en pression de ladite charge, suivie du mélange d'une fraction de ladite charge, comprimée, avec l'effluent organique riche en alcool issu de l'étape h) et avec une fraction de l'effluent aqueux issu de l'étape f), puis préchauffe dudit mélange par échange de chaleur, avantageusement avec un effluent oléfinique issu de l'étape g), avec un effluent aqueux provenant de l'étape f) et avec un effluent de déshydratation issu de l'étape c) de manière à produire une charge partiellement vaporisée, la fraction résiduelle de charge comprimée étant envoyée vers l'étape h) ;

b) Vaporisation et surchauffe finale de ladite charge partiellement vaporisée à une température comprise entre 250 et 375°C de manière à produire une charge vaporisée ;

c) Déshydratation de ladite charge vaporisée dans au moins un réacteur de déshydratation opérant en phase gaz à une température moyenne pondérée comprise entre 250 et 375°C, à une pression comprise entre 0,2 MPa et 1 MPa et à une PPH comprise entre 1 et 18 h$^{-1}$, en présence d'un catalyseur comprenant une zéolithe présentant au moins une série de canaux dont l'ouverture est définie par un anneau à 8 atomes d'oxygène (8MR), ledit catalyseur étant préalablement coké in-situ ou ex-situ, de manière à produire un effluent de déshydratation ;

d) Refroidissement dudit effluent de déshydratation issu de l'étape c) à une température comprise entre 30 et 50°C, avantageusement par au moins quatre échanges de chaleur indirects successifs avec au moins ladite charge de l'étape a), les phases organique et aqueuses issues de l'étape e) et une utilité froide, de manière à produire un effluent refroidi ;

e) Séparation par décantation dudit effluent refroidi en une phase aqueuse et une phase organique ;

f) Séparation par distillation de ladite phase aqueuse issue de l'étape e) de manière à produire un effluent hydrocarbures légers et un effluent aqueux, une fraction dudit effluent aqueux étant purgée, l'autre partie étant recyclée vers l'étape a) ;

g) Séparation par distillation de ladite phase organique issue de l'étape e) de manière à produire un effluent oléfinique et un effluent hydrocarbures lourds ;

h) Mélange de l'effluent hydrocarbures lourds issu de l'étape g) avec la fraction résiduelle de charge comprimée issue de l'étape a), le débit de ladite fraction résiduelle étant ajusté de manière à ce que le ratio massique des débits de ladite fraction résiduelle sur alcool non-converti dans ledit effluent hydrocarbures lourds soit compris entre 2:1 et 10:1, puis séparation par distillation dudit mélange de manière à produire un effluent hydrocarbures $C_5$-$C_6$, un effluent organique riche en alcool et un effluent hydrocarbures $C_6^+$, et recyclage dudit effluent organique riche en alcool vers l'étape a).

Par effluent hydrocarbures légers, on entend un effluent comprenant majoritairement des hydrocarbures comprenant au plus 4 atomes de carbone, c'est-à-dire au moins 50% poids d'hydrocarbures comprenant au plus 4 atomes de carbone.

Par effluent oléfinique, on entend un effluent comprenant au moins 50% poids avantageusement au moins 70% poids, préférentiellement au moins 80% poids, d'oléfines correspondantes à ou aux alcools primaires substitués en position 2 par un groupement alkyle alimentés dans la charge du procédé selon l'invention.

Par effluent hydrocarbures lourds, on entend un effluent comprenant au moins 50% poids d'hydrocarbures comprenant au moins 4 atomes de carbone, avantageusement au moins 5 atomes de carbone.

Le procédé selon l'invention vise à produire principalement des oléfines linéaires dans un effluent oléfinique issu de l'étape g), lesdites oléfines linéaires étant les oléfines correspondant à ou aux alcools primaires substitués en position 2 par un groupement alkyle alimentés dans la charge du procédé selon l'invention, et à recycler l'alcool non-converti obtenu suite à une dilution d'une phase hydrocarbures lourds par l'alcool de la charge et une distillation dudit mélange.

Un avantage du procédé selon l'invention est, par une dilution de l'effluent Hydrocarbures Lourds issu de l'étape g), d'améliorer la récupération de l'alcool non réagi en améliorant la pureté de l'effluent organique riche en alcool recyclé.

Outre une meilleure récupération, cet arrangement limite l'accumulation d'impuretés dans la boucle de recyclage, ce qui évite ou diminue les purges nécessaires sur lesdites boucles, améliorant donc le rendement global du procédé.

## DESCRIPTION DÉTAILLÉE DE L'INVENTION

Conformément à l'invention, le procédé selon l'invention est un procédé de déshydratation isomérisante d'une charge comprenant de 40 à 100% poids d'alcool primaire substitué en position 2 par un groupement alkyl.

La charge traitée dans le procédé selon l'invention est une charge comprenant au moins un alcool, avantageusement au moins un monoalcool primaire de formule R-CH$_2$-OH, dans lequel R est un radical alkyl non linéaire de formule générale $C_nH_{2n+1}$ où n est un entier compris entre 3 et 20, de préférence entre 3 et 10, de manière préférée entre 3 et 5.

La charge comprend de 40 à 100% poids, préférentiellement de 70 à 100% poids, de manière avantageuse de 85 à 100% poids d'au moins un alcool, avantageusement d'au moins un monoalcool primaire tel que défini précédemment.

Parmi les monoalcools primaires utilisables dans le procédé selon l'invention, on peut citer l'isobutanol, le 2-methylbutan-1-ol, le 2,2-dimethylpropan-1-ol, le 2-methylpentan-1-ol, le 2,2-dimethylbutan-1-ol, le 2-ethylbutan-1-ol. Ladite charge peut comprendre un ou plusieurs monoalcools primaires.

Ledit monoalcool primaire est préférentiellement l'isobutanol ou le 2-methyl-1-butanol, pris seul ou en mélange. Très préférentiellement, ledit monoalcool primaire est l'isobutanol.

Ladite charge peut provenir de procédés chimiques ou biochimiques, par exemple fermentaires. En particulier, cette charge peut être issue de procédés de fermentation de biomasse lignocellulosique.

Ladite charge peut contenir de l'eau, avantageusement jusqu'à 15% poids d'eau, préférentiellement jusqu'à 10% poids, très avantageusement jusqu'à 5% poids. Elle peut également comprendre des impuretés de type minéral (telles que Na, Ca, P, Al, Si, K, SO$_4$) et de type organique (telles que du méthanol, de l'éthanol, du n-butanol, des aldéhydes, des cétones, et les acides correspondant, par exemple l'acide furanique, acétique, isobutyrique).

## Étape a) de préchauffe

Conformément à l'invention, le procédé selon l'invention comprend une étape de mise en pression de ladite charge, suivie du mélange d'une fraction de ladite charge, comprimée, avec l'effluent organique riche en alcool issu de l'étape h) et avec une fraction de l'effluent aqueux issu de l'étape f), puis préchauffe dudit mélange par échange de chaleur, avantageusement avec un effluent oléfinique issu de l'étape g), avec un effluent aqueux provenant de l'étape f) et avec un effluent de déshydratation issu de l'étape c) de manière à produire une charge partiellement vaporisée, la fraction résiduelle de charge comprimée étant envoyée vers l'étape h).

La mise en pression peut être réalisée par tout moyen connu de l'homme du métier, en particulier à l'aide d'une pompe.

Ladite charge est mise en pression à une pression comprise entre 0,2 et 1 MPa. Ladite préchauffe permet audit mélange d'atteindre une température comprise entre 100 et 250°C, préférentiellement entre 100 et 200°C.

La fraction résiduelle de charge comprimée est envoyée vers l'étape h) en mélange avec l'effluent hydrocarbures lourds issu de l'étape g), le débit de ladite fraction résiduelle étant ajustée de manière à ce que le ratio massique des débits fraction résiduelle sur alcool non-converti dans un effluent hydrocarbures lourds soit compris entre 2:1 et 10:1.

Par débit alcool non-converti dans un effluent hydrocarbures lourds, on entend le débit dans l'effluent hydrocarbures lourds issu de l'étape g) de mono-alcool primaire de formule R-CH$_2$-OH, dans lequel R est un radical alkyl non linéaire de formule générale C$_n$H$_{2n+1}$ où n est un entier compris entre 3 et 20, de préférence entre 3 et 10, de manière préférée entre 3 et 5, préférentiellement un alcool choisi dans le groupe constitué par l'isobutanol, le 2-methylbutan-1-ol, le 2,2-dimethylpropan-1-ol, le 2-methylpentan-1-ol, le 2,2-dimethylbutan-1-ol, le 2-ethylbutan-1-ol et leurs mélanges, très préférentiellement dans le groupe constitué par l'isobutanol, le 2-methyl-1-butanol et leurs mélanges et de manière très préféré l'isobutanol.

**Étape b) de vaporisation et de surchauffe**

Conformément à l'invention, le procédé selon l'invention comprend une étape b) de vaporisation et de surchauffe finale de ladite charge partiellement vaporisée issue de l'étape a) de manière à produire une charge vaporisée.

De manière avantageuse, ladite charge partiellement vaporisée issue de l'étape a) est vaporisée par au moins un échange indirect avec une utilité chaude, puis surchauffé par un échange indirect avec une utilité chaude. Ladite charge est surchauffé jusqu'à une température comprise entre 250 et 375°C, préférentiellement entre 310 et 365°C.

**Étape c) de déshydratation**

Conformément à l'invention, le procédé selon l'invention comprend une étape de déshydratation de ladite charge vaporisée dans au moins un réacteur de déshydratation opérant en phase gaz à une température moyenne pondérée comprise entre 250 et 375°C, à une pression comprise entre 0,2 MPa et 1 MPa et à une PPH comprise entre 1 et 18 h-1, en présence d'un catalyseur comprenant une zéolithe présentant au moins une série de canaux dont l'ouverture est définie par un anneau à 8 atomes d'oxygène (8MR), ledit catalyseur étant préalablement coké in-situ ou ex-situ, de manière à produire un effluent de déshydratation.

L'étape de déshydratation comprend au moins un réacteur de déshydratation. Lorsque cette étape comprend plus d'un réacteur, la température à l'entrée de chacun des réacteurs est ajustée à une valeur comprise entre 250 et 375°C par un moyen de chauffe, la réaction de déshydratation isomérisante étant endothermique, et chaque réacteur est opéré dans des conditions identiques. Ainsi, dans la suite de l'exposé, le terme « le réacteur » désigne aussi bien le réacteur de ladite étape c), lorsque celle-ci ne comprend qu'un réacteur, que chacun des réacteurs de ladite étape c), lorsque celle-ci comprend plus d'un réacteur.

Le réacteur est opéré en phase gaz, à une température moyenne pondérée comprise entre 250 et 375°C, à une pression comprise entre 0,2 MPa et 1 MPa, à une PPH comprise entre 1 et 18 h-1, en présence d'un catalyseur comprenant une zéolithe comprenant au moins une série de canaux dont l'ouverture est définie par un anneau à 8 atomes d'oxygène (8MR). Ledit catalyseur est disposé dans un ou plusieurs lits fixes, lesquels peuvent être opérés en écoulement ascendant, descendant ou radial.

Par PPH, on entend « Poids par Poids par Heure », c'est-à-dire le débit massique d'alcool primaire substitué en position 2 par un groupement alkyl dans la charge en entrée de réacteur divisé par la masse de catalyseur dans ledit réacteur. Cette notion est également parfois désignée sous son acronyme anglais de WHSV, ou « Weight Hourly Space Velocity ».

Par température moyenne pondérée, on entend la moyenne de la température dans le lit catalytique calculée le long de l'axe d'écoulement dans ledit lit. Soit un lit de longueur L et de surface S, le mélange réactif s'écoulant le long de l'axe longitudinal x de ce lit, l'entrée dans le lit catalytique formant l'origine de l'axe (x=0), la température moyenne pondérée s'exprime selon :

$$\mathrm{TMP} = \frac{1}{L}\int_0^L T(x)dx$$

Conformément à l'invention, le catalyseur mis en œuvre dans ladite étape réactionnelle de déshydratation comprend une zéolithe présentant au moins une série de canaux dont l'ouverture est définie par un anneau à 8 atomes d'oxygène (8MR) telle que définie dans la classification "Atlas of Zeolite Structure Types", Ch. Baerlocher, L. B. Mc Cusker, D.H. Olson, 6ème Edition, Elsevier, 2007, Elsevier".

Selon un mode de réalisation particulier, la zéolithe peut également avantageusement contenir au moins une série de canaux dont l'ouverture de pores est définie par un anneau contenant 10 atomes d'oxygène (10 MR).

Ladite zéolithe est avantageusement choisie parmi les zéolithes ayant des canaux 8 et 10MR telles que les zéolithes de type structural FER et MFS, prises seules ou en mélange. La zéolite est plus avantageusement choisie dans le groupe constitué par, pour le type FER, les zéolithes ferrierite, FU-9, ISI-6, NU-23, ZSM-35 et pour le type MFS, la zéolite ZSM-57, et leurs mélanges. Ladite zéolithe est très avantageusement de type FER, et de préférence la ferrierite. De préférence, ladite zéolite est constituée de ferrierite.

De façon préférée, ladite zéolite est une ferrierite de rapport molaire Si/Al de 8 à 70, de préférence 10 à 70, de préférence choisi entre 10 et 50, de manière préférée choisi entre 20 et 50.

Ledit catalyseur comprend également un liant.

La teneur en zéolite dans le catalyseur est de 55-90%pds, de préférence entre 60 et 80%pds.

Le liant est avantageusement choisi parmi un liant silicique, un AlPO4, une argile, une zircone, un oxyde de Ti, du SiC. De façon très préférée, c'est un liant silicique.

La teneur en liant dans le catalyseur est comprise entre 10 et 45% pds, de préférence entre 20 et 40%. Le catalyseur peut éventuellement contenir des impuretés en faible quantité n'ayant pas d'effet technique sur la conversion /sélectivité du catalyseur. Ledit catalyseur peut être mis en forme par toutes les techniques connues de l'Homme du métier, par exemple sous forme de poudre, de billes, de pastilles, de granulés, ou d'extrudés (cylindres creux ou non, cylindres multilobés à 2, 3, 4 ou 5 lobes par exemple, cylindres torsadés), d'anneaux, etc...

Généralement, le catalyseur ne comprend pas de métaux. On comprend par cette expression « pas de métaux », qu'il n'y a pas de métaux ajoutés lors de la préparation. On comprend également qu'il peut y avoir des impuretés dans les liants et donc en faibles quantités. De manière préférée, il n'y a pas d'aluminium ou de fer dans la silice.

Ladite étape réactionnelle c) est une étape dans laquelle l'alcool est déshydraté en oléfine, les alcools branchés étant avantageusement déshydratés en oléfines linéaires. Ladite étape réactionnelle du procédé selon l'invention est avantageusement une étape de déshydratation isomérisante.

**Étape d) de refroidissement**

Conformément à l'invention, le procédé selon l'invention comprend une étape de refroidissement dudit effluent de déshydratation, avantageusement par au moins quatre échanges de chaleur indirects successifs avec au moins ladite charge issue de l'étape a), les phases organique et aqueuse issues de l'étape e) et une utilité froide, de manière à produire un effluent refroidi.

Ladite étape de refroidissement permet de refroidir ledit effluent de déshydratation à une température comprise entre 30 et 50°C, préférentiellement entre 35 et 45°C. Cette température de refroidissement permet d'une part de liquéfier l'effluent de déshydratation, et d'autre part d'aboutir à la démixtion de l'effluent liquéfié en une phase aqueuse et une phase organique, cette fourchette de température permettant de maximiser la récupération d'alcène et d'alcool.

**Étape e) de décantation**

Conformément à l'invention, le procédé selon l'invention comprend une étape de séparation par décantation dudit effluent refroidi issu de l'étape d) en une phase aqueuse et une phase organique.

Cette étape peut être mise en œuvre par tout moyen connu de l'Homme du métier, par exemple dans un ballon de décantation, la phase aqueuse étant soutirée en partie inférieure et la phase organique en partie supérieure.

**Étape f) de traitement d'une phase aqueuse**

Conformément à l'invention, le procédé selon l'invention comprend une étape de séparation par distillation de ladite phase aqueuse issue de l'étape e) de manière à produire un effluent hydrocarbures légers et un effluent aqueux, une fraction dudit effluent aqueux étant purgée, l'autre partie étant recyclée vers l'étape a).

La fraction de l'effluent aqueux recyclé vers l'étape a) représente entre 10 et 20% poids du débit de la phase aqueuse issue de l'étape e), avantageusement entre 12 et 17% poids. Ladite étape de séparation est opérée à une pression comprise entre 0,8 et 0,9 MPa.

La fraction de l'effluent aqueux recyclé vers l'étape a) est ajustée de manière à ce que la teneur en eau dans la charge vaporisée à l'entrée de l'étape c) soit comprise entre 4 et 15% poids, avantageusement entre 4 et 10% poids, très avantageusement entre 5 et 10% poids.

**Étape g) de séparation**

Conformément à l'invention, le procédé selon l'invention comprend une étape de séparation par distillation de la phase organique issue de l'étape e) de manière à produire un effluent alcènes et un effluent hydrocarbures lourds.

Ladite distillation est mise en œuvre dans au moins une colonne à distiller, opérée à une pression comprise entre 0,6 et 0,7 MPa. Cette distillation, connue de l'Homme du métier, est opérée selon les règles de l'art.

**Étape h) de séparation de l'alcool non-converti et de recyclage d'alcool**

Conformément à l'invention, le procédé selon l'invention comprend une étape comprenant le mélange de l'effluent hydrocarbures lourds issu de l'étape g) avec la fraction résiduelle de charge comprimée issue de l'étape a), le débit de ladite fraction résiduelle étant ajusté de manière à ce que le ratio massique des débits de fraction résiduelle sur alcool non-converti dans un effluent hydrocarbures lourds soit compris entre 2:1 et 10:1, puis la séparation par distillation dudit mélange de manière à produire un effluent hydrocarbures C5-C6, un effluent organique riche en alcool et un effluent hydrocarbures C6+, et recyclage dudit effluent organique riche en alcool vers l'étape a).

Le ratio massique des débits de fraction résiduelle sur alcool non-converti dans effluent hydrocarbures lourds est avantageusement compris entre 5:1 et 9:1. Cette dilution permet d'obtenir un effluent organique riche en alcool comprenant entre 47% et 92% mol d'alcool, préférentiellement entre 79% et 91% mol d'alcool et une récupération globale d'alcool sur cette étape (ratio du débit massique d'alcool dans l'effluent organique riche en alcool sur le débit massique d'alcool dans l'effluent hydrocarbures lourds) comprise entre 92 et 96%, avantageusement entre 94 et 96%.

La dilution de l'effluent hydrocarbures lourds avec une fraction de la charge est contre-intuitive, car elle conduit à priori à la pollution d'une fraction de la charge par la mise en contact avec un effluent comprenant de nombreuses impuretés alors que cette charge devrait être directement alimentée dans l'étape de réaction, après préchauffe et vaporisation. Or il a été observé que l'accroissement observé dans la pureté de l'effluent organique riche en alcool permettait de compenser cet effet de pollution, d'une part par une amélioration de la récupération de l'alcool non réagit, et d'autre part par la diminution des purges nécessaires sur la boucle de recyclage dudit effluent organique riche en alcool.

EXEMPLES

Exemple 1 (non-conforme) : recyclage de l'isobutanol sans dilution par l'isobutanol de la charge

Un effluent hydrocarbures lourds alimente une colonne de distillation dans laquelle il est séparé en un effluent organique riche en isobutanol, extrait par un soutirage latéral, en un effluent hydrocarbures C5-C6 en tête et en un effluent hydrocarbures C6+ en fond. Le débit dudit soutirage latéral est ajusté pour extraire 95% de l'isobutanol alimentant la colonne. La composition dudit soutirage latéral est présentée dans le Tableau 1.

Tableau 1 - Composition de l'effluent organique riche en isobutanol

| %molaire | |
|---|---|
| Isobutanol | 22.65 |
| Acide acétique | 11.37 |
| 2-méthyl-2-butène | 0.77 |
| Isobutaldéhyde | 0.07 |
| n-butanol | 0.10 |
| s-butanol | 0.38 |
| t-butanol | 0.05 |
| 2,3,3-triméthyl-1-butène | 0.25 |
| 2,4,4-triméthyl-1-pentène | 63.02 |

**Exemple 2: recyclage de l'isobutanol avec la dilution 2/1 par l'isobutanol de la charge**

*Cet exemple diffère de l'exemple 1 en ce que l'effluent hydrocarbures lourds est dilué par une fraction de la charge avant d'être séparé.*

Un effluent hydrocarbures lourds alimente une colonne de distillation. Préalablement à son alimentation, il est dilué avec une fraction de la charge du procédé avec un ratio de débits massiques effluent fraction de charge / isobutanol dans hydrocarbures lourds égal à 2/1. Ce mélange est séparé en un effluent organique riche en isobutanol, extrait par un soutirage latéral, en un effluent hydrocarbures C5-C6 en tête et en un effluent hydrocarbures C6+ en fond. Le débit dudit soutirage latéral est ajusté pour extraire 95% de l'isobutanol alimentant la colonne. La composition dudit soutirage est présentée dans le Tableau 2.

Tableau 2 - Composition de l'effluent organique riche en isobutanol

| %molaire | |
|---|---|
| Isobutanol | 47.18 |
| Acide acétique | 5.76 |
| 2-méthyl-2-butène | 0.68 |
| Isobutaldéhyde | 0.06 |
| n-butanol | 0.08 |
| s-butanol | 0.24 |
| t-butanol | 0.04 |
| 2,3,3-triméthyl-1-butène | 0.79 |
| 2,4,4-triméthyl-1-pentène | 44.28 |

On observe que la concentration en isobutanol dans le soutirage latéral est améliorée par rapport à l'exemple 1.

**Exemple 3: recyclage de l'isobutanol avec la dilution 10/1 par l'isobutanol de la charge**

*Cet exemple diffère de l'exemple 1 en ce que l'effluent hydrocarbures lourds est dilué par une fraction de la charge avant d'être séparé.*

Un effluent hydrocarbures lourds alimente une colonne de distillation. Préalablement à son alimentation, il est dilué avec une fraction de la charge du procédé avec un ratio de débits massiques fraction de charge / isobutanol dans effluent hydrocarbures lourds égal à 10/1. Ce mélange est séparé en un effluent organique riche en isobutanol, extrait par un soutirage latérale, en un effluent hydrocarbures C5-C6 en tête et en un effluent hydrocarbures C6+ en fond. Le débit dudit soutirage latéral est ajusté pour extraire 95% de l'isobutanol compris dans l'alimentation de la colonne. La composition dudit soutirage est présentée dans le Tableau 3.

Tableau 3 - Composition de l'effluent organique riche en isobutanol

| %molaire | |
|---|---|
| Isobutanol | 92.39 |
| Acide acétique | 1.14 |
| 2-méthyl-2-butène | 0.40 |
| Isobutaldéhyde | 0.04 |
| n-butanol | 0.03 |
| s-butanol | 0.14 |
| t-butanol | 0.02 |
| 2,3,3-triméthyl-1-butène | 0.27 |
| 2,4,4-triméthyl-1-pentène | 3.37 |

On observe que la concentration en isobutanol dans le soutirage latéral est améliorée par rapport à l'exemple 1.

## Revendications

1. Procédé de déshydratation isomérisante d'une charge comprenant de 40 à 100% poids d'alcool primaire substitué en position 2 par un groupement alkyl comprenant au moins les étapes suivantes :

   a) Mise en pression de ladite charge, suivie du mélange d'une fraction de ladite charge, comprimée, avec l'effluent organique riche en alcool issu de l'étape h) et avec une fraction de l'effluent aqueux issu de l'étape f), puis préchauffe dudit mélange, de manière à produire une charge partiellement vaporisée, la fraction résiduelle de charge comprimée étant envoyée vers l'étape h) ;
   b) Vaporisation et surchauffe finale de ladite charge partiellement vaporisée à une température comprise entre 250 et 375°C de manière à produire une charge vaporisée ;
   c) Déshydratation de ladite charge vaporisée dans au moins un réacteur de déshydratation opérant en phase gaz à une température moyenne pondérée comprise entre 250 et 375°C, à une pression comprise entre 0,2 MPa et 1 MPa et à une PPH comprise entre 1 et 18 h-1, en présence d'un catalyseur comprenant une zéolithe présentant au moins une série de canaux dont l'ouverture est définie par un anneau à 8 atomes d'oxygène (8MR), ledit catalyseur étant préalablement coké in-situ ou ex-situ, de manière à produire un effluent de déshydratation ;
   d) Refroidissement dudit effluent de déshydratation issu de l'étape c) à une température comprise entre 30 et 50°C, de manière à produire un effluent refroidi ;
   e) Séparation par décantation dudit effluent refroidi en une phase aqueuse et une phase organique ;
   f) Séparation par distillation de ladite phase aqueuse issue de l'étape e) de manière à produire un effluent hydrocarbures légers comprenant au moins 50% poids d'hydrocarbures comprenant au plus 4 atomes de carbone et un effluent aqueux, une fraction dudit effluent aqueux étant purgée, l'autre partie étant recyclée vers l'étape a) ;
   g) Séparation par distillation de ladite phase organique issue de l'étape e) de manière à produire un effluent oléfinique comprenant au moins 50% poids d'oléfines correspondantes à ou aux alcools primaires substitués en position 2 par un groupement alkyle alimentés dans ladite charge et un effluent hydrocarbures lourds ;
   h) Mélange de l'effluent hydrocarbures lourds issu de l'étape g) avec la fraction résiduelle de charge comprimée issue de l'étape a), le débit de ladite fraction résiduelle étant ajusté de manière à ce que le ratio massique des débits de fraction résiduelle sur alcool non-converti dans un effluent hydrocarbures lourds soit compris entre 2:1 et 10:1, puis séparation par distillation dudit mélange de manière à produire un effluent hydrocarbures C5-C6, un effluent organique riche en alcool et un effluent hydrocarbures C6+, et recyclage dudit effluent organique riche en alcool vers l'étape a).

2. Procédé selon la revendication 1 dans lequel la préchauffe de ladite étape a) est réalisée par échange de chaleur avec un effluent oléfinique issu de l'étape g), avec un effluent aqueuse provenant de l'étape f) et avec un effluent de déshydratation issu de l'étape c).

3. Procédé selon l'une des revendications précédentes dans lequel ladite charge partiellement vaporisée est vaporisée et surchauffée dans ladite étape b) à une température comprise entre 310 et 365°C.

4. Procédé selon l'une des revendications précédentes dans lequel le catalyseur mis en œuvre dans l'étape c) comprend une zéolithe comprenant au moins une série de canaux dont l'ouverture de pores est définie par un anneau contenant 10 atomes d'oxygène.

5. Procédé selon l'une des revendications précédentes dans lequel le refroidissement selon l'étape d) est réalisé par au moins quatre échanges de chaleur indirects successifs avec au moins ladite charge de l'étape a), les phases organique et aqueuses issues de l'étape e) et une utilité froide.

6. Procédé selon l'une des revendications précédentes dans lequel le débit de ladite fraction résiduelle dans ladite étape h) est ajusté de manière à ce que le ratio massique des débits de fraction résiduelle sur alcool non-converti dans un effluent hydrocarbures lourds sur soit compris entre 5:1 et 9:1.

**Patentansprüche**

1. Verfahren zur isomerisierenden Dehydratisierung eines Einsatzstoffs, der 40 bis 100 Gew.-% in 2-Position durch eine Alkylgruppe substituierten primären Alkohols umfasst, wobei das Verfahren mindestens

   a) Druckbeaufschlagen des Einsatzstoffs und anschließendes Mischen eines Teils des komprimierten Ausgangsstoffs mit dem alkoholreichen organischen Austragsstrom aus Schritt h) und mit einem Teil des wässrigen Austragsstroms aus Schritt f), dann Vorerhitzen der Mischung zur Bildung eines teilweise verdampften Einsatzstoffs, wobei der restliche Teil des komprimierten Einsatzstoffs Schritt h) zugeführt wird;
   b) Verdampfen und schließliches Überhitzen des teilweise verdampften Einsatzstoffs bei einer Temperatur zwischen 250 und 375 °C zur Bildung eines verdampften Einsatzstoffs;
   c) Dehydratisieren des verdampften Einsatzstoffs in mindestens einem bei einer gewichteten mittleren Temperatur zwischen 250 und 375 °C, bei einem Druck zwischen 0,2 MPa und 1 MPa und bei einem GGS zwischen 1 und 18 h$^{-1}$ arbeitenden Dehydratisierungsreaktor in Gegenwart eines Katalysators, der einen Zeolith umfasst, der mindestens eine Reihe von Kanälen aufweist, deren Öffnung durch einen Ring mit 8 Sauerstoffatomen (8MR) definiert ist, wobei der Katalysator vorher in situ oder ex situ verkokt wird, zur Bildung eines Dehydratisierungsaustragsstroms;
   d) Abkühlen des Dehydratisierungsaustragsstroms aus Schritt c) auf eine Temperatur zwischen 30 und 50 °C zur Bildung eines abgekühlten Austragsstroms;
   e) Trennen des Ausgangsstoffs durch Absetzenlassen in eine wässrige Phase und eine organische Phase;
   f) Trennen der wässrigen Phase aus Schritt e) durch Destillation zur Bildung eines Austragsstroms von leichten Kohlenwasserstoffen, der mindestens 50 Gew.-% Kohlenwasserstoffe mit höchstens 4 Kohlenstoffatomen umfasst, und eines wässrigen Austragsstroms, wobei ein Teil des wässrigen Austragsstroms ausgeschleust und der andere Teil zu Schritt a) zurückgeführt wird;
   g) Trennen der organischen Phase aus Schritt e) durch Destillation zur Bildung eines olefinischen Austragsstroms, der mindestens 50 Gew.-% Olefine, die dem bzw. den in 2-Position durch eine Alkylgrupe substituierten primären Alkoholen, die in den Einsatzstoff eingespeist werden, umfasst, und eines Austragsstroms von schweren Kohlenwasserstoffen;
   h) Mischen des Austragsstroms von schweren Kohlenwasserstoffen aus Schritt g) mit dem restlichen Teil des komprimierten Einsatzstoffs aus Schritt a), wobei der Durchsatz des restlichen Teils so eingestellt wird, dass das Durchsatz-Massenverhältnis von restlichem Teil zu nicht umgesetztem Alkohol in einem Austragsstrom von schweren Kohlenwasserstoffen zwischen 2:1 und 10:1 liegt, dann Trennung der Mischung durch Destillation zur Bildung eines Austragsstroms von C5-C6-Kohlenwasserstoffen, eines alkoholreichen organischen Austragsstroms und eines Austragsstroms von C6+-Kohlenwasserstoffen und Rückführen des alkoholreichen organischen Austragsstroms zu Schritt a) umfasst.

2. Verfahren nach Anspruch 1, wobei das Vorerhitzen von Schritt a) durch Wärmeaustausch mit einem olefinischen Austragsstrom aus Schritt g), mit einem wässrigen Austragsstrom aus Schritt f) und mit einem Dehydratisierungsaustragsstrom aus Schritt c) durchgeführt wird.

3. Verfahren nach einem der vorhergehenden Ansprüche, wobei der teilweise verdampfte Einsatzstoff in Schritt b) bei einer Temperatur zwischen 310 und 365 °C verdampft und überhitzt wird.

4. Verfahren nach einem der vorhergehenden Ansprüche, wobei der in Schritt c) eingesetzte Katalysator einen Zeolith umfasst, der mindestens eine Reihe von Kanälen aufweist, deren Öffnung durch einen Ring mit 10 Sauerstoffatomen definiert ist.

5. Verfahren nach einem der vorhergehenden Ansprüche, wobei das Abkühlen gemäß Schritt d) durch mindestens vier aufeinanderfolgende indirekte Wärmetäusche mit mindestens dem Einsatzstoff aus Schritt a), der organischen und wässrigen Phase aus Schritt e) und einem kalten Medium durchgeführt wird.

**6.** Verfahren nach einem der vorhergehenden Ansprüche, wobei der Durchsatz des restlichen Teils in Schritt h) so eingestellt wird, dass das Durchsatz-Massenverhältnis von restlichem Teil zu nicht umgesetztem Alkohol in einem Austragsstrom von schweren Kohlenwasserstoffen zu zwischen 5:1 und 9:1 liegt.

**Claims**

**1.** Process for the isomerizing dehydration of a feedstock comprising from 40% to 100% by weight of primary alcohol substituted in position 2 with an alkyl group, comprising at least the following steps:

a) placing said feedstock under pressure, followed by mixing a fraction of said compressed feedstock with the alcohol-rich organic effluent obtained from step h) and with a fraction of the aqueous effluent obtained from step f), followed by preheating said mixture, so as to produce a partially vaporized feedstock, the residual fraction of compressed feedstock being sent into step h);
b) vaporization and final superheating of said partially vaporized feedstock to a temperature of between 250 and 375°C so as to produce a vaporized feedstock;
c) dehydration of said vaporized feedstock in at least one dehydration reactor operating in the gas phase at a weighted mean temperature of between 250 and 375°C, at a pressure of between 0.2 MPa and 1 MPa and at a WHSV of between 1 and 18 $h^{-1}$, in the presence of a catalyst comprising a zeolite containing at least one series of channels whose aperture is defined by a ring containing 8 oxygen atoms (8MR), said catalyst being coked *in-situ* or *ex-situ* beforehand, so as to produce a dehydration effluent;
d) cooling of said dehydration effluent obtained from step c) to a temperature of between 30 and 50°C, so as to produce a cooled effluent;
e) separation by settling of said cooled effluent as an aqueous phase and an organic phase;
f) separation by distillation of said aqueous phase obtained from step e) so as to produce a light hydrocarbon effluent comprising at least 50% by weight of hydrocarbons comprising not more than 4 carbon atoms and an aqueous effluent, a fraction of said aqueous effluent being purged, the other part being recycled into step a);
g) separation by distillation of said organic phase obtained from step e) so as to produce an olefinic effluent comprising at least 50% by weight of olefins corresponding to primary alcohol(s) substituted in position 2 with an alkyl group, fed into said feedstock, and a heavy hydrocarbon effluent;
h) mixing of the heavy hydrocarbon effluent obtained from step g) with the residual fraction of compressed feedstock obtained from step a), the flow rate of said residual fraction being adjusted so that the mass ratio of the flows of residual fraction to unconverted alcohol in a heavy hydrocarbon effluent is between 2:1 and 10:1, followed by separation by distillation of said mixture so as to produce a C5-C6 hydrocarbon effluent, an alcohol-rich organic effluent and a C6+ hydrocarbon effluent, and recycling of said alcohol-rich organic effluent into step a).

**2.** Process according to Claim 1, in which the preheating of said step a) is performed by heat exchange with an olefinic effluent obtained from step g), with an aqueous effluent originating from step f) and with a dehydration effluent obtained from step c).

**3.** Process according to either of the preceding claims, in which said partially vaporized feedstock is vaporized and superheated in said step b) to a temperature of between 310 and 365°C.

**4.** Process according to one of the preceding claims, in which the catalyst used in step c) comprises a zeolite comprising at least one series of channels whose pore aperture is defined by a ring containing 10 oxygen atoms.

**5.** Process according to one of the preceding claims, in which the cooling in step d) is performed by means of at least four successive indirect heat exchanges with at least said feedstock from step a), the organic and aqueous phases obtained from step e) and a cold utility.

**6.** Process according to one of the preceding claims, in which the flow rate of said residual fraction in said step h) is adjusted so that the mass ratio of the flows of residual fraction to unconverted alcohol in a heavy hydrocarbon effluent to is between 5:1 and 9:1.

## RÉFÉRENCES CITÉES DANS LA DESCRIPTION

**Documents brevets cités dans la description**

- EP 2348005 A
- WO 2011113834 A
- WO 2016046242 A

**Littérature non-brevet citée dans la description**

- **CH. BAERLOCHER ; L. B. MC CUSKER ; D.H. OLSON.** Atlas of Zeolite Structure Types. Elsevier, 2007